# EUROPEAN PATENT APPLICATION

(11) **EP 4 796 531 A1**
(43) Date of publication of application: **26.08.2026**
(21) Application number: 24879407.5
(22) Date of filing: 01.08.2024
(51) Int. Cl.: C07C 315/04, C07C 37/52, C07C 39/08, C07C 39/15, C07C 39/16, C07C 45/61, C07C 49/84, C07C 317/22, C08J 11/10

(54) **METHOD FOR DECOMPOSING SYNTHETIC RESIN**

(30) Priority: 20.10.2023 JP 2023180795
(71) Applicant: National Institute of Advanced Industrial Science and Technology, Chiyoda-ku Tokyo 100-8921 (JP)
(72) Inventor: MINAMI, Yasunori, Tsukuba-shi, Ibaraki 305-8560 (JP); YOSHIDA, Masaru, Tsukuba-shi, Ibaraki 305-8560 (JP); SATO, Kazuhiko, Tsukuba-shi, Ibaraki 305-8560 (JP)
(74) Representative: Curell Suñol S.L.P.
(86) International application number: PCT/JP2024/027626
(87) International publication number: WO 2025/083977

(57) **Abstract**

[Object] To provide a synthetic resin decomposition method for a synthetic resin containing at least one of polysulfone, polyphenylsulfone, polyethersulfone, polyether ether sulfone, and polyether ether ketone.

[Solution] A synthetic resin decomposition method mixes: a synthetic resin; a base; at least one of methanol, ethanol, isopropyl alcohol, tert-butyl alcohol, butanol, pentanol, hexanol, heptanol, octanol, nonanol, decanol, 2-ethylhexanol benzyl alcohol, 2-phenylethyl alcohol, phenol, cresol, 4-tert-butylphenol, 4-methoxyphenol, hydroquinone, bisphenol A (2,2-bis(4-hydroxyphenyl)propane), and bisphenol S (bis(4-hydroxyphenyl)sulfone); and an organic solvent.

## Description

### Technical Field

The present invention relates to a synthetic resin decomposition method for a synthetic resin containing at least one of polysulfone, polyphenylsulfone, polyethersulfone, polyether ether sulfone, and polyether ether ketone.

### Background Art

Among super engineering plastics widely used as stable materials, polysulfone (PSU) is a thermoplastic resin which not only excels in heat resistance, a wide use temperature range, hydrolysis resistance, chemical resistance, and electrical properties, but also possesses ideal characteristics as a processable material, as having mechanical strength, transparency, moldability, and weather resistance. As having these characteristics, polysulfone is widely used in various applications, such as medical and food processing fields including medical biomembrane substitutes, artificial kidneys, medical instruments, food containers, cooking appliance parts, and the like, and electronic device components such as connectors and printed circuit boards.

Additionally, super engineering plastics similar to polysulfone include polyphenylsulfone (PPSU), polyethersulfone (PES), polyether ether sulfone (PEES), and polyether ether ketone (PEEK), all of which have excellent stability and characteristics.

As of 2019, the total production volume of polysulfone and polyphenylsulfone was 26,600 tons and the production volume of polyether ether ketone was approximately 7,000 tons, which appear to be low. Considering that these synthetic resins are expensive, their production volume cannot be considered low. Given their excellent characteristics mentioned above, they are essential materials for the industrial society of the future.

On the other hand, disposal and recycling of these synthetic resins is difficult because of their high stability. These synthetic resins have molecular structures containing many benzene rings, making them difficult to be decomposed even by microorganisms. In addition, these synthetic resins are used in high-priced products, whereby disposal of them under current conditions results in a significant economic loss.

Therefore, with regard to chemical recycling of super engineering plastics, there have been proposals, which are a report on reduction of polysulfone molecular weight (Patent Literature 1), examples of decomposing polyphenylene sulfide into monomer units and oligomer units at temperatures of 150°C or higher (Patent Literature 2, Non-Patent Literatures 1 to 5), and decomposition of polyethersulfone using subcritical water (Patent Literature 3).

Further, recently, there have been reports on depolymerization of polyether ether ketone with a sulfur nucleophile at 150°C (Non-Patent Literature 6), and depolymerization of polysulfone, polyether ketone, polyphenylsulfone, polyether ether sulfone, and polyether ether ketone with an alkali hydroxide at 150°C (Non-Patent Literature 7).

### Citation List

### Patent Literature

Patent Literature 1: International Publication No. WO2008/004642
Patent Literature 2: Japanese Unexamined Patent Application Publication No. 2013-249324
Patent Literature 3: Japanese Unexamined Patent Application Publication No. 2022-170457

### Non-Patent Literature

Non-Patent Literature 1: Yu, Z. L.; Miao, G. X.; Chen, Y. R. Macromol Chem Phys 1996, 197, 4061.
Non-Patent Literature 2: Wang, S. J.; Bian, S. G.; Yan, H.; Xiao, M.; Meng, Y. Z. J. App. Poly. Sci. 2008, 110, 4049.
Non-Patent Literature 3: Lian, Z.; Bhawal, B. N.; Morandi, B. Science 2017, 356, 1059.
Non-Patent Literature 4: Minami, Y.; Matsuyama, N.; Matsuo, Y.; Tamura, M.; Sato, K.; Nakajima, Y. Synthesis 2021, 53, 3351.
Non-Patent Literature 5: Delcaillau, T.; Woenckhaus-Alvarez, A.; Morandi, B. Org. Lett. 2021, 23, 7018.
Non-Patent Literature 6: Minami, Y.; Matsuyama, N.; Takeichi, Y.; Watanabe, R.; Mathew, S.; Nakajima, Y. Communications Chemistry, 2023, 6, 14.
Non-Patent Literature 7: Minami, Y.; Inagaki, Y.; Tsuyuki, T.; Sato, K.; Nakajima, JACS Au DOI: 10.1021/jacsau.3c00357.
Non-Patent Literature 8: Shigeno, M.; Hayashi, K.; Nozawa-Kumada, K.; Kondo, Y. Org. Lett. 2019, 21, 5505.

### Summary of Invention

### Technical Problem

However, although some methods for depolymerizing super engineering plastics have been developed as mentioned above, they are fragmented technologies and require relatively high temperature conditions (higher than 120°C). The development of chemical recycling technology for super engineering plastics has just begun, and there is a need to develop depolymerization methods (decomposition methods) based on various ideas and to improve the value of resulting products.

Regarding reactions related to the method for decomposing super engineering plastics, a substitution reaction of the methoxy group of para-methoxy substituted benzophenone, which is one of the structural units of polyphenylene sulfide, with an amino group using organic superbase catalyst t-Bu-P4 and arylamine is known (Non-Patent Literature 8). However, there are no known examples of this method being applied to the depolymerization of polysulfone, polyphenylsulfone, or polyethersulfone.

In light of the circumstances described above, the present invention aims to provide a synthetic resin decomposition method for a synthetic resin containing at least one of polysulfone, polyphenylsulfone, polyethersulfone, polyether ether sulfone, and polyether ether ketone.

Note that these issues do not preclude the existence of other issues. In addition, it is not necessary for each aspect of the present invention described below to solve all of these problems (issues). Furthermore, other problems (issues) can be extracted from the description of the specification, drawings, or claims.

### Solution to Problem

The inventors of the present invention have continued to conduct extensive research into the above-mentioned problems and have discovered the following revolutionary synthetic resin decomposition method for a synthetic resin containing at least one of polysulfone, polyphenylsulfone, polyethersulfone, polyether ether sulfone, and polyether ether ketone.

A first aspect of the present invention to solve the above-mentioned issues is a synthetic resin decomposition method for a synthetic resin containing at least one of polysulfone, polyphenylsulfone, polyethersulfone, polyether ether sulfone, and polyether ether ketone. The synthetic resin decomposition method includes a decomposition step for mixing the synthetic resin, a base, an alcohol, the alcohol being at least one of methanol, ethanol, isopropyl alcohol, tert-butyl alcohol, butanol, pentanol, hexanol, heptanol, octanol, nonanol, decanol, 2-ethylhexanol, benzyl alcohol, 2-phenylethyl alcohol, phenol, cresol, 4-tert-butylphenol, 4-methoxyphenol, hydroquinone, bisphenol A (2,2-bis(4-hydroxyphenyl)propane), and bisphenol S (bis(4-hydroxyphenyl)sulfone), and an organic solvent so as to decompose the polysulfone, the polyphenylsulfone, the polyethersulfone, the polyether ether sulfone, and the polyether ether ketone, contained in the synthetic resin.

According to the first aspect, compared to conventional decomposition methods, a synthetic resin can be decomposed quickly at a low reaction temperature (in a case of fatty acid alcohols).

A second aspect according to the present invention is the synthetic resin decomposition method according to the first aspect, in which the base includes at least one of sodium hydroxide, potassium hydroxide, potassium phosphate, cesium hydroxide, cesium carbonate, sodium tert-butoxide, lithium tert-butoxide, potassium tert-butoxide, lithium bis(trimethylsilyl)amide, sodium bis(trimethylsilyl)amide, and potassium bis(trimethylsilyl)amide.

According to the second aspect, a synthetic resin can be decomposed more quickly.

A third aspect according to the present invention is the synthetic resin decomposition method according to the first or second aspect, in which the organic solvent includes at least one of 1,3-dimethyl-2-imidazolidinone, tetramethylurea, 1,3-dimethylpropyleneurea, N-methyl-2-pyrrolidone, N,N-dimethylacetamide, dimethyl sulfoxide, and tetrahydrothiophene-1,1-dioxide.

According to the third aspect, a synthetic resin can be decomposed still more quickly.

A fourth aspect according to the present invention is the synthetic resin decomposition method according to the first aspect, in which a reaction temperature in the decomposition step is within a range of 25°C to 200°C.

According to the fourth aspect, a synthetic resin can be decomposed at a lower reaction temperature.

A fifth aspect according to the present invention is the synthetic resin decomposition method according to the first aspect, in which a reaction temperature in the decomposition step is within a range of 25°C to 120°C.

According to the fifth aspect, a synthetic resin can be decomposed at a still lower reaction temperature.

A sixth aspect according to the present invention is the synthetic resin decomposition method according to the first aspect, in which the synthetic resin contains at least one of polyether ether ketone, polyether ether sulfone, polysulfone, and polyethersulfone, the alcohol is at least one of hydroquinone, bisphenol A, and bisphenol S, and a reaction temperature in the reaction step is within a range of 25°C to 160°C.

According to the sixth aspect, monomers constituting polymer compounds contained in the synthetic resin can be recovered simply by cooling a reaction solution obtained in the present aspect, then adding hydrochloric acid thereto, and washing the obtained solid with water. Here, the reaction temperature in the present aspect is preferably within a range of 140°C to 160°C, as this allows for greater recovery of the monomers constituting the polymer compounds contained in the synthetic resin. In addition, the monomers can be used when resynthesizing the polymer compounds contained in the synthetic resin.

For example, hydroquinone is used as the alcohol when the synthetic resin contains at least one of polyether ether ketone and polyether ether sulfone, bisphenol A is used as the alcohol when the synthetic resin contains polysulfone, or bisphenol S is used as the alcohol when the synthetic resin contains polyethersulfone, and the reaction temperature in the reaction step is set within a range of 25°C to 160°C, and the synthetic resin decomposition method of the first aspect is applied, thereby enabling the recovery of monomers constituting the polymer compounds contained in each synthetic resin.

Further, for example, hydroquinone is used as the alcohol when the synthetic resin contains polysulfone, and the reaction temperature in the reaction step is set within the range of 25°C to 160°C, and the synthetic resin decomposition method of the first aspect is applied, thereby enabling the recovery of monomers constituting polymer compounds different from the polymer compounds contained in the synthetic resin.

Here, the contents of Japanese Patent Application No. 2023-180795 are hereby incorporated herein by reference as forming part of this specification.

### Brief Description of Drawings

[Fig. 1] Fig. 1 is a table related to a method for recovering synthetic resin decomposition products.
[Fig. 2] Fig. 2 is a chemical reaction formula for a reaction in Example 1.
[Fig. 3] Fig. 3 is a table showing reaction conditions in Example 1 and yields of respective synthetic resin decomposition products obtained.
[Fig. 4] Fig. 4 is a chemical reaction formula for a reaction in Example 2.
[Fig. 5] Fig. 5 is a chemical reaction formula for a reaction in Example 3.
[Fig. 6] Fig. 6 is a chemical reaction formula for a reaction in Example 4.
[Fig. 7] Fig. 7 is a chemical reaction formula for a reaction in Example 5.
[Fig. 8] Fig. 8 is a chemical reaction formula for reactions in Examples 6 to 8.
[Fig. 9] Fig. 9 is a table showing reaction conditions in Examples 6 to 8 and yields of respective synthetic resin decomposition products obtained.
[Fig. 10] Fig. 10 is a chemical reaction formula for a reaction in Example 9.
[Fig. 11] Fig. 11 is a chemical reaction formula for a reaction in Example 10.
[Fig. 12] Fig. 12 is a chemical reaction formula for a reaction in Example 11.
[Fig. 13] Fig. 13 is a chemical reaction formula for a reaction in Example 12.
[Fig. 14] Fig. 14 is a chemical reaction formula for a reaction in Example 13.
[Fig. 15] Fig. 15 is a graph showing a ¹H NMR analysis result of a product obtained in Example 13.
[Fig. 16] Fig. 16 is a chemical reaction formula for a reaction in Example 14.
[Fig. 17] Fig. 17 is a graph showing a ¹H NMR analysis result of a product obtained in Example 14.

### Description of Embodiments

Referring to the accompanying drawings, a description will be provided below on an embodiment of a synthetic resin decomposition method according to the present invention for synthetic resin containing at least one of polysulfone, polyphenylsulfone, polyethersulfone, polyether ether sulfone, and polyether ether ketone. However, note that the present invention is not limited to the following embodiment.

### (Embodiment 1)

### <<Synthetic resin decomposition method>>

A synthetic resin decomposition method according to the present embodiment includes a decomposition step for mixing (blending) at least one of methanol, ethanol, isopropyl alcohol, tert-butyl alcohol, butanol, pentanol, hexanol, heptanol, octanol, nonanol, decanol, 2-ethylhexanol benzyl alcohol, 2-phenylethyl alcohol, phenol, cresol, 4-tert-butylphenol, 4-methoxyphenol, hydroquinone, bisphenol A (2,2-bis(4-hydroxyphenyl)propane), and bisphenol S (bis(4-hydroxyphenyl)sulfone), with a synthetic resin, a base, and an organic solvent, to decompose polysulfone, polyphenylsulfone, polyethersulfone, polyether ether sulfone, or polyether ether ketone contained in the synthetic resin.

According to the synthetic resin decomposition method of the present embodiment, compared to conventional decomposition methods, a synthetic resin can be decomposed quickly at a low reaction temperature (for example, 25°C to 120°C in a case of fatty acid alcohols, and 150°C to 180°C in a case of aromatic alcohols).

### <Synthetic resin>

A synthetic resin which is a decomposition target of the synthetic resin decomposition method according to the present embodiment (target synthetic resin) is not particularly limited as long as the synthetic resin contains at least one of polysulfone, polyphenylsulfone, polyethersulfone, polyether ether sulfone, and polyether ether ketone. The synthetic resin may contain a plurality of polymer compounds among these, but preferably contains a single polymer compound (synthetic polymer compound), and more preferably contains a single polymer compound at high concentration (preferably 70% to 100%, more preferably 80% to 100%, and particularly preferably 90% to 100%). Using the synthetic resin containing a single polymer compound of the ones allows for efficient (quick) decomposition and, as described later, recovery of only predetermined bisphenol or hydroquinone with a high yield. Further, using the synthetic resin containing a single polymer compound at high concentration allows for efficient (quick) decomposition and, as described later, recovery of only predetermined bisphenol or hydroquinone with a higher yield.

Here, polysulfone is a synthetic polymer compound with the following repeating structure.

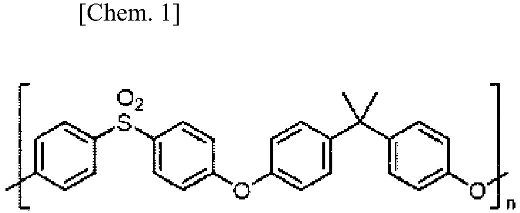

Here, n is within a range of 20 to 300, preferably within a range of 30 to 250 for enabling easy decomposition, and more preferably within a range of 35 to 200 for enabling particularly easy decomposition.

Further, polyphenylsulfone is a synthetic polymer compound with the following repeating structure.

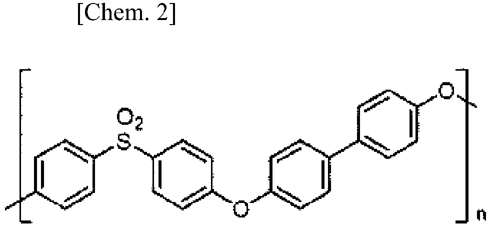

Here, n is within a range of 25 to 140, preferably within a range of 35 to 120 for enabling easy decomposition, and more preferably within a range of 40 to 110 for enabling particularly easy decomposition.

Polyethersulfone is a synthetic polymer compound with the following repeating structure.

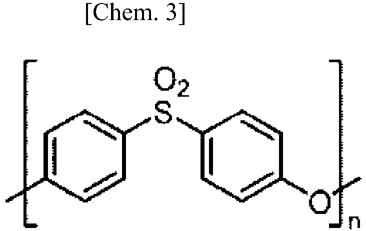

Here, n is within a range of 40 to 180, preferably within a range of 55 to 150 for enabling easy decomposition, and more preferably within a range of 65 to 140 for enabling particularly easy decomposition.

Polyether ether sulfone is a synthetic polymer compound with the following repeating structure.

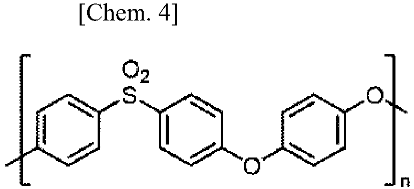

Here, n is within a range of 10 to 300, preferably within a range of 15 to 200 for enabling easy decomposition, and more preferably within a range of 20 to 150 for enabling particularly easy decomposition.

Polyether ether ketone is a synthetic polymer compound with the following repeating structure.

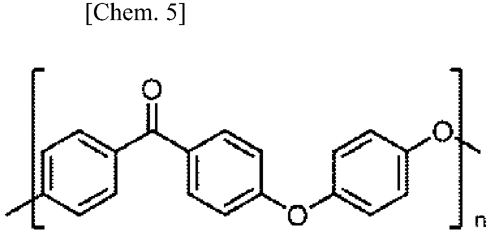

Here, n is within a range of 25 to 100, preferably within a range of 30 to 85 for enabling easy decomposition, and more preferably within a range of 38 to 73 for enabling particularly easy decomposition.

The synthetic resin may contain components other than polysulfone, polyphenylsulfone, polyethersulfone, polyether ether sulfone, and polyether ether ketone. Specifically, the synthetic resin may contain polyester, polyamide, polycarbonate, polyfluorocarbon, and the like. Even though these are contained, according to the synthetic resin decomposition method of the present invention, polysulfone, polyphenylsulfone, polyethersulfone, polyether ether sulfone, or polyether ether ketone contained in a synthetic resin can be decomposed.

### <Alcohol>

In the synthetic resin decomposition method according to the present embodiment, alcohol is not particularly limited as long as the alcohol contains at least one of methanol, ethanol, isopropyl alcohol, tert-butyl alcohol, butanol, pentanol, hexanol, heptanol, octanol, nonanol, decanol, 2-ethylhexanol benzyl alcohol, 2-phenylethyl alcohol, phenol, cresol, 4-tert-butylphenol, 4-methoxyphenol, hydroquinone, bisphenol A (2,2-bis(4-hydroxyphenyl)propane), and bisphenol S (bis(4-hydroxyphenyl)sulfone). These alcohols are considered to possess sufficient nucleophilicity toward polysulfone, polyphenylsulfone, polyethersulfone, polyether ether sulfone, and polyether ether ketone when they react with bases to form alkoxides.

Further, among these alcohols, methanol is particularly preferred. Using methanol allows for quicker decomposition of synthetic resins compared to conventional decomposition methods. Furthermore, among the phenols, hydroquinone is preferred. Using hydroquinone allows the above-mentioned renewable monomer to be obtained with a higher yield compared to other phenols.

The amount of alcohol used in the synthetic resin decomposition method according to the present embodiment is not particularly limited, but it is preferable to react alcohol with the synthetic resin in an amount of 2 to 16 equivalents, more preferably 4 to 14 equivalents, and particularly preferably 6 to 12 equivalents.

### <Base>

In the synthetic resin decomposition method according to the present embodiment, a base is not particularly limited, but is preferably one capable of activating the above-mentioned alcohol to produce an alkoxide. Specific examples of the base include sodium hydroxide, potassium hydroxide, potassium phosphate, cesium hydroxide, cesium carbonate, sodium tert-butoxide, lithium tert-butoxide, potassium tert-butoxide, lithium bis(trimethylsilyl)amide, sodium bis(trimethylsilyl)amide, and potassium bis(trimethylsilyl)amide. Using these bases allows for easier decomposition of synthetic resins.

In the synthetic resin decomposition method according to the present embodiment, the amount of base is not particularly limited, but it is preferable to react the base with the synthetic resin in an amount of 2 to 4 equivalents, more preferably 2 to 3 equivalents, and particularly preferably 2 to 2.4 equivalents.

### <Organic solvent>

In the synthetic resin decomposition method according to the present embodiment, the organic solvent that can be used is not particularly limited, but 1,3-dimethyl-2-imidazolidinone, tetramethylurea, 1,3-dimethylpropyleneurea, N-methyl-2-pyrrolidone, N,N-dimethylacetamide, dimethyl sulfoxide, tetrahydrothiophene-1,1-dioxide, and the like are preferred, and 1,3-dimethyl-2-imidazolidinone and 1,3-dimethylpropyleneurea are particularly preferred. These organic solvents can improve the solubility of the salt produced by the reaction between the above alcohol and the above base through solvation, can maintain the nucleophilicity of the produced alkoxide, are less likely to react with the alkoxide (1,3-dimethyl-2-imidazolidinone and 1,3-dimethylpropyleneurea are particularly stable toward alkoxide), and can be used even at high temperatures (100°C to 200°C). Using these organic solvents allows for easier decomposition of synthetic resins.

The amount of the organic solvent used in the synthetic resin decomposition method according to the present embodiment is not particularly limited, but it is preferable to react the organic solvent with the synthetic resin in an amount of 0.1 to 11 moles, more preferably 0.3 to 4 moles, and particularly preferably 0.5 to 1 moles, relative to mole which is a monomer unit of the synthetic resin.

### <Reaction condition in decomposition step>

The synthetic resin decomposition according to the present embodiment can be performed by mixing at least one of methanol, ethanol, isopropyl alcohol, tert-butyl alcohol, butanol, pentanol, hexanol, heptanol, octanol, nonanol, decanol, 2-ethylhexanol benzyl alcohol, 2-phenylethyl alcohol, phenol, cresol, 4-tert-butylphenol, 4-methoxyphenol, hydroquinone, bisphenol A (2,2-bis(4-hydroxyphenyl)propane), and bisphenol S (bis(4-hydroxyphenyl)sulfone) with the synthetic resin, a base, and an organic solvent.

The order of mixing (blending) these materials (substances) is not particularly limited, but it is preferable to prepare a raw material composition which is a blend of an alcohol, a base, and an organic solvent, and then mix this raw material composition with a synthetic resin. By adopting such a mixing order, the decomposition of the synthetic resin can proceed at a higher rate.

The reaction temperature during the synthetic resin decomposition (reaction temperature in the decomposition step) can be appropriately adjusted taking into account the synthetic polymer compound contained in the synthetic resin, and the decomposition may be performed at room temperature (25°C) or under heating conditions. Specifically, the reaction temperature during the synthetic resin decomposition can be within a range of 25°C to 200°C. However, it is preferable that the reaction temperature during the synthetic resin decomposition is within a range of 25°C to 180°C because the synthetic resin can be easily decomposed. Furthermore, when an aliphatic alcohol is used as the alcohol, it is preferable that the reaction temperature during the synthetic resin decomposition is within a range of 25°C to 120°C because the synthetic resin can be more easily decomposed, and it is more preferable that the reaction temperature during the synthetic resin decomposition is within a range of 60°C to 100°C because the synthetic resin can be easily and quickly decomposed. In this specification, aliphatic alcohol refers to methanol, ethanol, isopropyl alcohol, tert-butyl alcohol, butanol, pentanol, hexanol, heptanol, octanol, nonanol, decanol, and 2-ethylhexanol benzyl alcohol.

On the other hand, when an aromatic phenol is used as the alcohol, it is preferable that the reaction temperature during the synthetic resin decomposition is within a range of 120°C to 160°C because the synthetic resin can be more easily decomposed, and it is more preferable that the reaction temperature during the synthetic resin decomposition is within a range of 140°C to 160°C because the synthetic resin can be easily and quickly decomposed. In this specification, aromatic phenol refers to 2-phenylethyl alcohol, phenol, cresol, 4-tert-butylphenol, 4-methoxyphenol, hydroquinone, bisphenol A (2,2-bis(4-hydroxyphenyl)propane), and bisphenol S (bis(4-hydroxyphenyl)sulfone).

Here, a device used for heating is not particularly limited, and a device such as a heater can be used.

Further, the reaction time during the synthetic resin decomposition (reaction time in the decomposition step) is preferably 1 to 40 hours, and more preferably 15 to 24 hours. If the reaction time is shorter than 1 hour, the synthetic resin cannot be sufficiently decomposed, and if it is longer than 40 hours, a side reaction occurs which decomposes a carbon group derived from alcohol. Furthermore, during the synthetic resin decomposition, it is preferable to stir a reaction mixture containing all of the above-mentioned materials by known means. Stirring the reaction mixture allows for quicker decomposition of synthetic resins.

In addition, the synthetic resin decomposition according to the present embodiment can be performed under an atmosphere of an inert gas (preferably with a concentration of 99% or higher) or air. Examples of the inert gas include rare gases such as argon, and nitrogen.

### <<Method for recovering synthetic resin decomposition product>>

A synthetic resin decomposition product produced by the synthetic resin decomposition method according to the present embodiment contains a predetermined bisphenol and a predetermined bisphenol ether. Therefore, by employing this synthetic resin decomposition method, bisphenol A can be recovered from synthetic resins containing polysulfone, and 4,4'-dihydroxybiphenyl can be recovered from synthetic resins containing polyphenylsulfone and polyether ether sulfone. Here, bisphenol ether can ultimately yield a predetermined bisphenol through a known hydrolysis reaction.

Furthermore, after employing the synthetic resin decomposition method according to the present embodiment, hydroquinone can be recovered from synthetic resins containing polyether ether sulfone or polyether ether ketone by employing, for example, column chromatography.

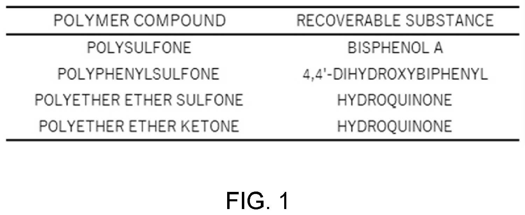

Specifically, by employing the synthetic resin decomposition method, it is possible to recover substances shown in the right column of the table in Fig. 1 from polymer compounds shown in the left column of the table. In this synthetic resin decomposition method, methanol or hydroquinone is used as the alcohol, and sodium hydroxide is used as the base.

Next, the synthetic resin decomposition method according to the present embodiment will be described in more detail with reference to examples, but the present invention is not limited to the following examples. The present invention includes combinations of synthetic resins, bases, alcohols, and organic solvents which are not described in the following examples. However, those skilled in the art can understand from the following examples that even combinations not described in the examples can similarly decompose synthetic resins, and can actually verify this through experiments.

### <Example 1>

Under an argon atmosphere, a base (2.2 equivalents used relative to polysulfone), each solvent (0.4 mL), and alcohol (methanol) were added sequentially to 88.5 mg of pellet-shaped polysulfone (PSU) (0.20 mmol, calculated based on the monomer molar weight, average Mw ~35000, average Mn ~16000, Cat. No. 428302 (manufactured by Sigma-Aldrich Co. LLC), and the obtained reaction mixture was then stirred while being heated at room temperature (25°C) to 100°C.

Thereafter, the temperature of the reaction mixture was brought to room temperature (25°C), and 1 M hydrochloric acid was added to quench the reaction. Next, ethyl acetate was added, and an organic layer was extracted from the obtained two-layer solution, dried over magnesium sulfate, and distilled under reduced pressure to obtain a crude product.

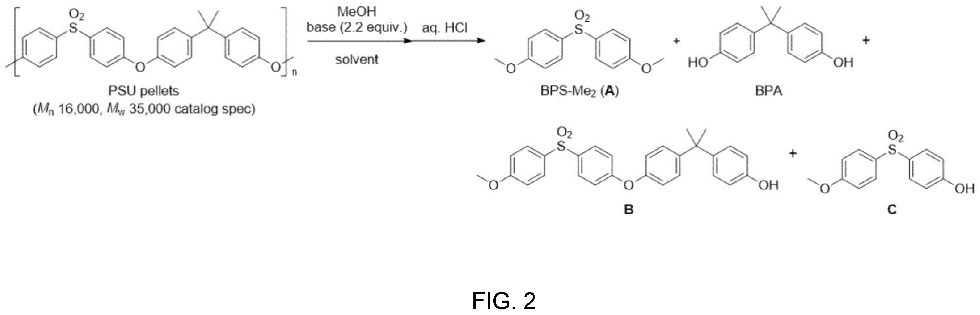

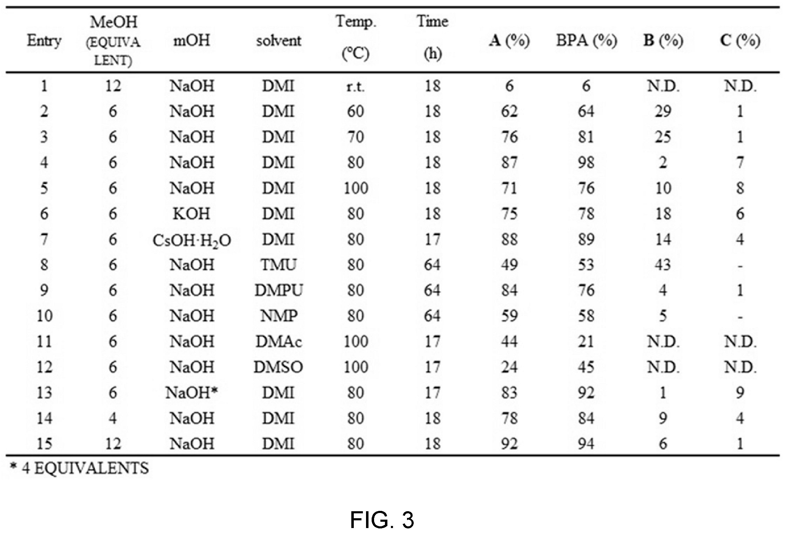

Then, the yields of bisphenol S dimethyl ether A (A), bisphenol A (BPA), its intermediate product B, and by-product C, which are obtained according to the chemical reaction formula shown in Fig. 2, were measured by ¹H NMR in deuterated chloroform using mesitylene as an internal standard. Fig. 3 shows reaction conditions of these and respective yields of obtained synthetic resin decomposition products.

In this drawing, DMI represents 1,3-dimethyl-2-imidazolidinone, TMU represents tetramethylurea, DMPU represents 1,3-dimethylpropyleneurea, NMP represents N-methyl-2-pyrrolidone, DMAc represents N,N-dimethylacetamide, and DMSO represents dimethyl sulfoxide. In addition, N.D. represents not detected, and r.t. represents room temperature (25°C).

As shown in this drawing, the decomposition reaction of polysulfone was performed using 4 to 12 equivalents of methanol at various temperatures (Entries 1 to 5), with various bases (Entries 4, 6, and 7) and organic solvents (Entries 4, 5, and 8 to 12).

As a result, it has been found that the decomposition (depolymerization) of the target polymer compound proceeds with high selectivity when using an inorganic strong base such as sodium hydroxide, potassium hydroxide, and cesium hydroxide, along with a urea-based solvent or amide-based solvent such as DMI, TMU, DMPU, NMP, and DMAc at a reaction temperature of 80°C.

In particular, it has been found that when the decomposition reaction (depolymerization) is conducted at 80°C using sodium hydroxide and DMI solvent, bisphenol S dimethyl ether A (A) and bisphenol A (BPA) can be obtained in high yields with almost no intermediate product B or by-product C produced (see Entry 4).

Furthermore, through examination of the amount of base (Entry 13) and the amount of methanol (Entries 14 and 15), it has been found that bisphenol S dimethyl ether A (A) and bisphenol A (BPA), which are the target synthetic resin decomposition products, can be obtained selectively and in high yield when using 2.2 equivalents of base and 12 equivalents of methanol (Entry 15).

### <Example 2>

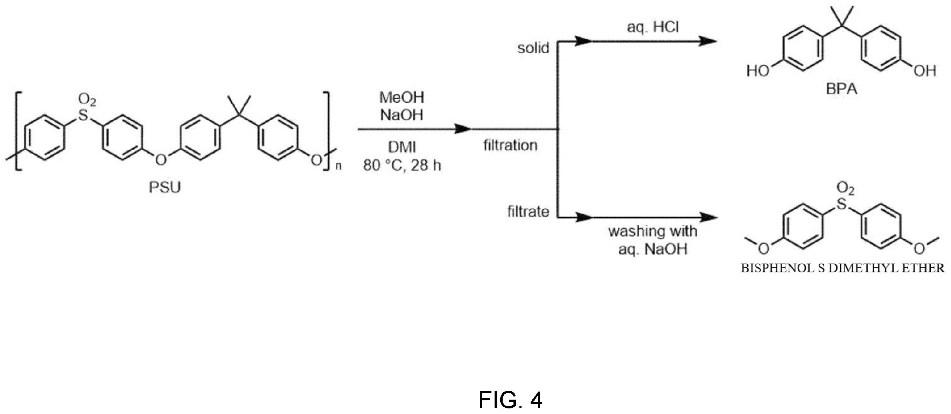

The chemical reaction shown in Fig. 4 was performed as follows.

Under an argon atmosphere, 1.25 mL of methanol, 226 mg (5.5 mmol) of sodium hydroxide, and 5 mL of 1,3-dimethyl-2-imidazolidinone (DMAc) were added sequentially to 1.10 g of colorless, transparent, pellet-shaped polysulfone (PSU) (2.49 mmol, calculated based on the monomer molar weight, average Mw ~35000, average Mn ~16000, Cat. No. 428302 (manufactured by Sigma-Aldrich Co. LLC), and the obtained reaction mixture was then stirred at 80°C.

After 28 hours, a yellow solution containing a white solid was brought to room temperature and then separated into the white solid and the yellow solution by filtration. Thereafter, an organic layer was extracted with ethyl acetate, dried over magnesium sulfate, and distilled under reduced pressure to obtain a crude product 1 containing bisphenol S dimethyl ether A.

Then, ethyl acetate and hydrochloric acid were added to the white solid obtained thus far to separate the layers, and the organic layer was then extracted, dried over magnesium sulfate, and distilled under reduced pressure to obtain a crude product containing bisphenol S dimethyl ether. Analysis by NMR revealed that crude product 2, containing mainly bisphenol A (BPA), was obtained.

Bisphenol S dimethyl ether A of the crude product 1 was purified to 421 mg (61%) by recrystallization using methylene chloride and hexane. Here, if necessary, the purity of bisphenol S dimethyl ether can be improved by silica gel column chromatography (changing the weight ratio of hexane:ethyl acetate from 95:5 to 6:4) (recovery amount 346 mg).

The crude product 2 was subjected to a base-acid extraction operation, specifically, ethyl acetate and a sodium hydroxide solution were added to the crude product 2 to convert bisphenol A into an anion, which was then dissolved in an aqueous layer, and hydrochloric acid and ethyl acetate were added to the extracted aqueous layer to regenerate bisphenol A, which was then dissolved in the organic layer. Further, this was dried over magnesium sulfate, and distilled under reduced pressure to obtain bisphenol A (380 mg, yield 66%).

The NMR measurement results of the obtained bisphenol S dimethyl ether and bisphenol A are shown below.

Bisphenol A: ¹H NMR (600 MHz, acetone-d₆) δ 1.58 (s, 6H, methyl), 6.72 (AA'BB', 4H, aromatic), 7.05 (AA'BB', 4H, aromatic), 8.07 (br, 2H, hydroxy).

Bisphenol S dimethyl ether: ¹H NMR (600 MHz, acetone-d₆) δ 3.87 (s, 6H, methyl), 7.09 (AA'BB', 4H, aromatic), 7.87 (AA'BB', 4H, aromatic). ¹³C NMR (151 MHz, acetone-d₆) δ 56.2, 115.4, 130.3, 135.4, 164.2.

### <Example 3>

A baby bottle made of polyphenylsulfone (PPSU) (ChuChu manufactured by JEX Co., Ltd.) was cut into 5 mm sides.

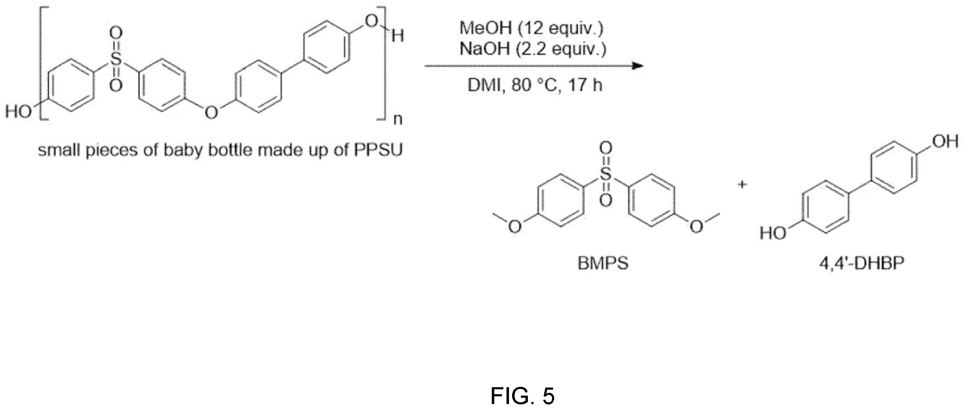

Under an argon atmosphere, 100 µL (2.4 mmol) of methanol, 17.6 mg (0.44 mmol) of sodium hydroxide, and 0.4 mL of 1,3-dimethyl-2-imidazolidinone (DMI) were added sequentially to the pale yellow polyphenylsulfone pieces mentioned-above (84.8 mg; an amount corresponding to 0.2 mmol of the repeating unit in the general formula shown on the right side of the chemical reaction formula in Fig. 5), and the obtained reaction mixture was then stirred at 80°C.

After 17 hours, a yellow solution containing a white solid was brought to room temperature (25°C) and then separated into the white solid and the yellow solution by filtration. Thereafter, ethyl acetate and hydrochloric acid were added to the separated white solid to separate the layers, and an organic layer was extracted. The organic layer was then dried over magnesium sulfate and distilled under reduced pressure to obtain 23.9 mg (0.128 mmol, 61%) of 4,4'-dihydroxybiphenyl.

Subsequently, the yellow solution was distilled under reduced pressure to obtain a crude product. The crude product thus obtained was subjected to preparative thin-layer silica gel chromatography (eluent: hexane/ethyl acetate 10/3) to obtain the target synthetic resin decomposition product, di(4-(methoxy)phenyl)sulfone (BMPS) (recovery amount 34.5 mg, 0.124 mmol, yield 59%).

The ¹H NMR and ¹³C NMR analysis results of the obtained di(4-(methoxy)phenyl)sulfone (BMPS) were found to be similar to those in Example 2.

The ¹H NMR analysis result of the obtained 4,4'-dihydroxybiphenyl is shown below.

4,4'-dihydroxybiphenyl: ¹H NMR (600 MHz, acetone-d₆) δ 6.87 (AA'BB', 4H, aromatic), 7.38 (AA'BB', 4H, aromatic).

### <Example 4>

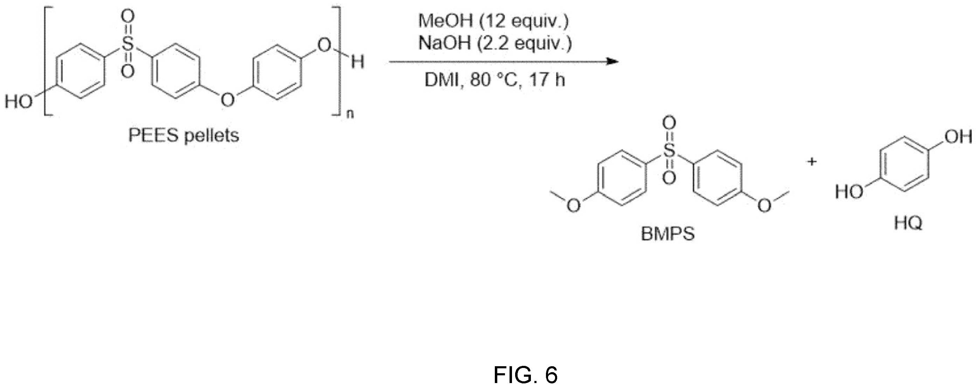

Under an argon atmosphere, 100 µL (2.4 mmol) of methanol, 18.1 mg (0.45 mmol) of sodium hydroxide, and 0.4 mL of 1,3-dimethyl-2-imidazolidinone (DMI) were added sequentially to brown pellet-shaped polyether ether sulfone (69.4 mg; an amount corresponding to 0.21 mmol of the repeating unit in the general formula on the right side of the chemical reaction formula in Fig. 6, manufactured by Sigma-Aldrich Co. LLC, Cat. No. 440965), and the obtained reaction mixture was then stirred at 80°C.

After 17 hours, a yellow solution containing a white solid was brought to room temperature (25°C) and then separated into the white solid and the yellow solution by filtration. The separated white solid was treated with hydrochloric acid, and ethyl acetate was added to further extract white solid.

Then, 1H NMR measurement was performed in deuterated chloroform with mesitylene as an internal standard. The result showed that hydroquinone (HQ) was produced with a yield of 34%.

Subsequently, hydrochloric acid was added to the yellow solution, and an organic layer was extracted with ethyl acetate, dried over magnesium sulfate, and distilled under reduced pressure to obtain a crude product.

The crude product thus obtained was subjected to preparative thin-layer silica gel chromatography (eluent: hexane/ethyl acetate 10/3) to obtain the target synthetic resin decomposition product, di(4-(methoxy)phenyl)sulfone (BMPS) (recovery amount 18.3 mg, 0.066 mmol, yield 31%).

The ¹H NMR and ¹³C NMR analysis results of the obtained di(4-(methoxy)phenyl)sulfone (BMPS) were found to be similar to those in Example 2.

The ¹H NMR analysis result of the produced hydroquinone is shown below.

Hydroquinone (HQ): ¹H NMR (600 MHz, acetone-d₆) δ 6.66 (s, 4H, aromatic).

### <Example 5>

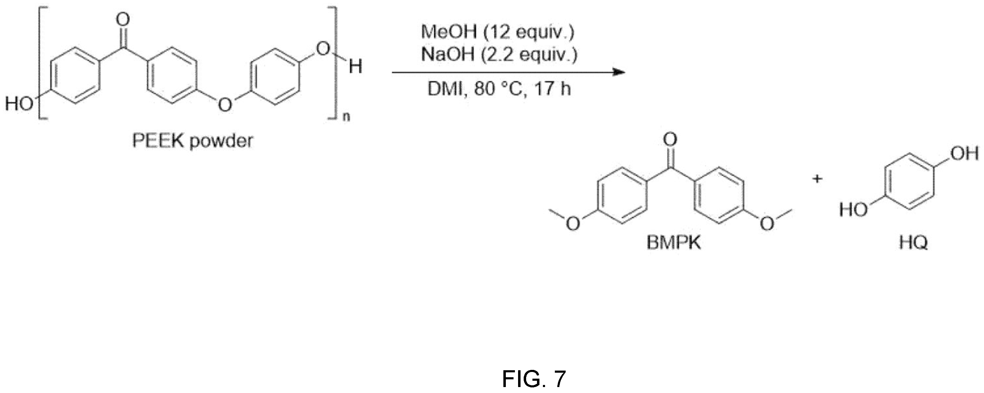

Under an argon atmosphere, 100 µL (2.4 mmol) of methanol, 19.5 mg (0.49 mmol) of sodium hydroxide, and 0.4 mL of 1,3-dimethyl-2-imidazolidinone (DMI) were added sequentially to powdered polyether ether ketone (57.5 mg; an amount corresponding to 0.20 mmol of the repeating unit in the general formula on the right side of the chemical reaction formula in Fig. 7, weight-average molecular weight 20800, number-average molecular weight 10300, manufactured by Sigma-Aldrich Co. LLC, Cat. No. 456640), and the mixture was then stirred at 80°C.

After 17 hours, a yellow solution containing a white solid was brought to room temperature (25°C) and then separated into the white solid and the yellow solution by filtration. The separated white solid was treated with hydrochloric acid, and ethyl acetate was added to further extract white solid.

Then, 1H NMR measurement was performed in deuterated chloroform with mesitylene as an internal standard. The result showed that hydroquinone (HQ) was produced with a yield of 42%. Hydroquinone can be recovered by known methods such as silica gel column chromatography.

Subsequently, hydrochloric acid was added to the yellow solution, and an organic layer was extracted with ethyl acetate, dried over magnesium sulfate, and distilled under reduced pressure to obtain a crude product.

The crude product thus obtained was subjected to preparative thin-layer silica gel chromatography (eluent: hexane/ethyl acetate 10/3) to obtain the target synthetic resin decomposition product, di(4-(methoxy)phenyl)ketone (BMPK) (recovery amount 27.7 mg, 0.114 mmol, yield 58%).

The ¹H NMR analysis result of the produced hydroquinone was found to be similar to that in Example 4.

The ¹H NMR analysis result of the obtained di(4-(methoxy)phenyl)ketone (BMPK) is shown below.

Di(4-(methoxy)phenyl)ketone (BMPK): ¹H NMR (600 MHz, acetone-d₆) δ 3.91 (s, 6H, methyl), 7.07 (AA'BB', 4H, aromatic), 7.76 (AA'BB', 4H, aromatic). ¹³C NMR (151 MHz, acetone-d₆) δ 55.9, 114.4, 131.6, 132.7, 163.9, 194.0.

### <Examples 6 to 8>

In the above-described examples, methanol was used as the alcohol, but the alcohol which can be used in the synthetic resin decomposition method according to the present embodiment is not limited to methanol.

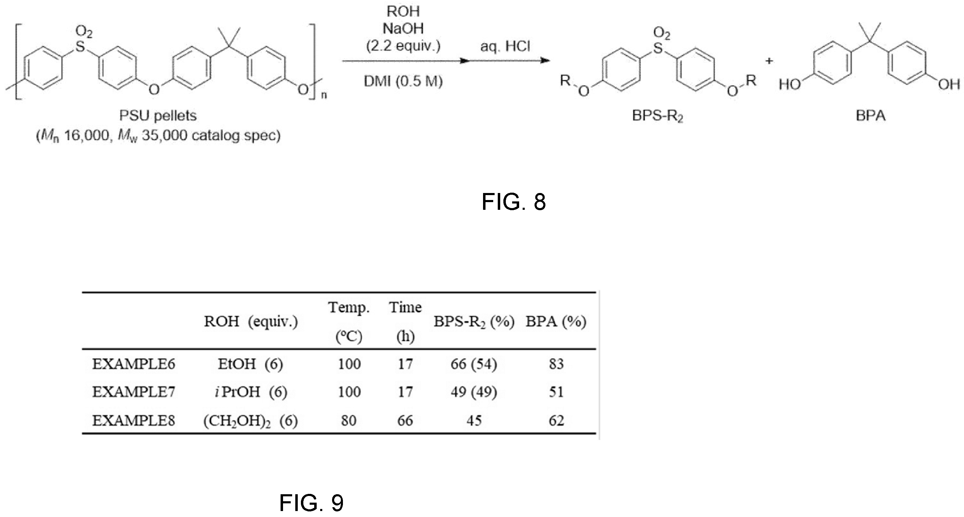

Fig. 9 shows reaction conditions and yields of synthetic resin decomposition products obtained when ethanol (Example 6), isopropyl alcohol (Example 7), and ethylene glycol (Example 8) were used as the alcohol in the chemical reaction formula shown in Fig. 8. Values listed in the ROH column of the table in this drawing (values enclosed in parentheses) indicate equivalent amounts of alcohol used in respective examples. For example, "EtOH (6)" indicates that 6 equivalents of ethanol were used. In addition, values in the column for the yield of BPS-R₂ in the table in this drawing indicate yields calculated by NMR, and values in parentheses next to them indicate yields when actually isolated. Furthermore, the BPA column in the table in this drawing indicates yields of bisphenol A (BPA).

As can be seen from this drawing, it was found that even when using ethanol, isopropyl alcohol, and ethylene glycol as alcohols, synthetic resins can be sufficiently decomposed.

### <Example 9>

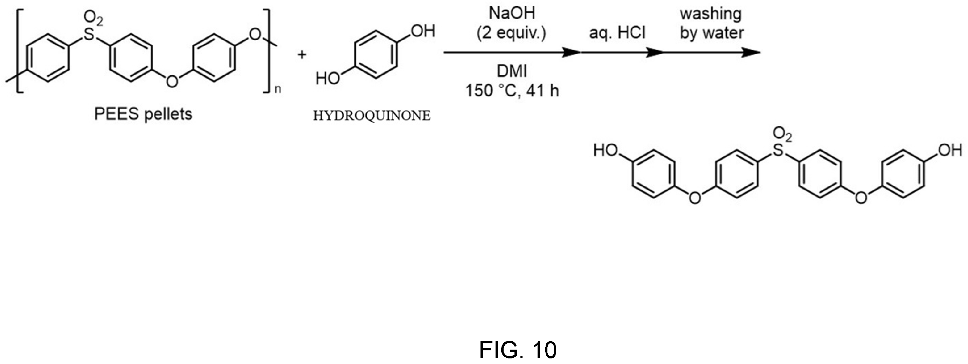

Under a nitrogen atmosphere, 488 mg (4.43 mmol) of hydroquinone, 321 mg (8.03 mmol) of sodium hydroxide, and 8.0 mL of 1,3-dimethyl-2-imidazolidinone (DMI) were added sequentially to brown pellet-shaped polyether ether sulfone (1.31 g; an amount corresponding to 4.01 mmol of the repeating unit in the general formula on the right side of the chemical reaction formula in Fig. 10, manufactured by Sigma-Aldrich Co. LLC, Cat. No. 440965), and the obtained reaction mixture was then stirred at 150°C.

After 41 hours, the reaction solution was brought to room temperature (25°C), hydrochloric acid was added, and an organic layer was extracted with ethyl acetate. Then, the organic layer was dried over magnesium sulfate and distilled under reduced pressure to obtain a crude product. Water was added to the crude product, which was then filtered, washed with water, and dried under reduced pressure to obtain the target synthetic resin decomposition product, 4,4'-((sulfonylbis(4,1-phenylene))bis(oxy))diphenol (recovery amount 1.55 g, 3.57 mmol, yield 89%).

The ¹H NMR and ¹³C NMR analysis results of the obtained 4,4'-((sulfonylbis(4,1-phenylene))bis(oxy))diphenol are shown below.

¹H NMR (600 MHz, acetone-d₆) δ 6.91 (AA'BB', 4H, aromatic), 6.98 (AA'BB', 4H, aromatic), 7.02 (AA'BB', 4H, aromatic), 8.49 (br, 2H, OH). ¹³C NMR (151 MHz, acetone-d₆) δ 117.4, 117.6, 122.8, 130.6, 136.4, 148.0, 155.8, 164.0.

### <Example 10>

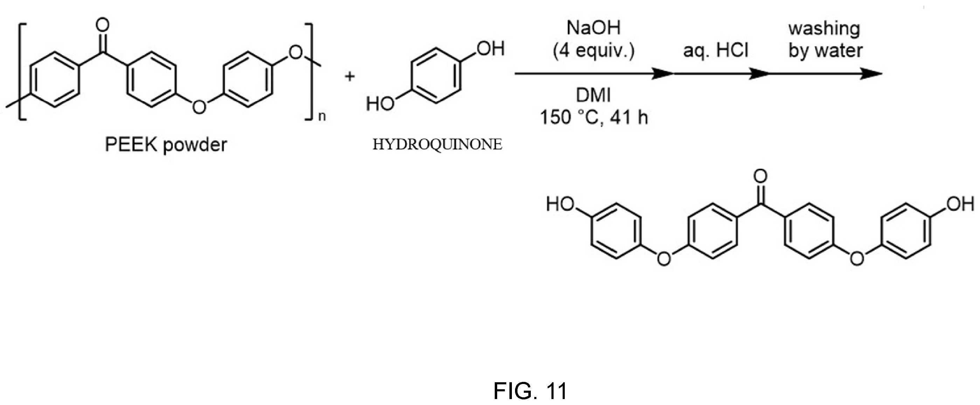

Under a nitrogen atmosphere, 1.61 g (14.6 mmol) of hydroquinone, 648 mg (16.2 mmol) of sodium hydroxide, and 8.0 mL of 1,3-dimethyl-2-imidazolidinone (DMI) were added sequentially to powdered polyether ether ketone (1.15 g; an amount corresponding to 4.00 mmol of the repeating unit in the general formula on the right side of the chemical reaction formula in Fig. 11, manufactured by Sigma-Aldrich Co. LLC, Cat. No. 456640), and the obtained reaction mixture was then stirred at 150°C.

After 41 hours, the reaction solution was brought to room temperature (25°C), hydrochloric acid was added, and the precipitated colorless solid was filtered to obtain a crude product. The crude product was washed with water, dried over magnesium sulfate, and then dried under reduced pressure to obtain the target synthetic resin decomposition product, bis(4-(4-hydroxyphenoxy)phenyl)methanone (recovery amount 1.37 g, 3.44 mmol, yield 86%). That is, in the synthetic resin decomposition method of the present example using hydroquinone, no operation for isolating column or the like is required, and bis(4-(4-hydroxyphenoxy)phenyl)methanone can be easily recovered simply by treating the crude product with hydrochloric acid and then washing the remaining solid with water.

The ¹H NMR and ¹³C NMR analysis results of the obtained bis(4-(4-hydroxyphenoxy)phenyl)methanone are shown below.

¹H NMR (600 MHz, acetone-d₆) δ 6.92 (AA'BB', 4H, aromatic), 7.00 (AA'BB', 4H, aromatic), 7.01 (AA'BB', 4H, aromatic), 7.78 (AA'BB', 4H, aromatic), 8.45 (br, 2H, hydroxy). ¹³C NMR (151 MHz, acetone-d₆) δ 116.8, 117.4, 122.7, 132.7, 132.9, 148.5, 155.6, 163.5, 193.9.

### <Example 11>

Carbon fiber reinforced polyether ether ketone (TECAPEEK CF30 manufactured by Ensinger GmbH, Cat. No. 3-3094-02) was processed into powder. The content ratio of polyether ether ketone relative to the total mass of this carbon fiber reinforced polyether ether ketone was 70 mass %.

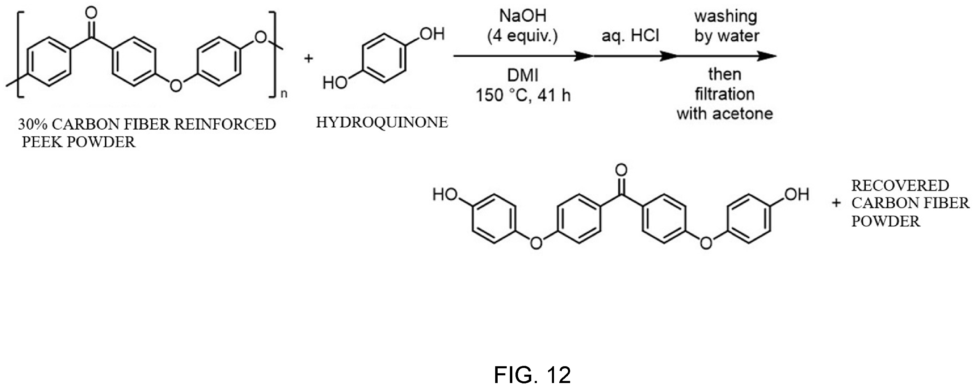

Under a nitrogen atmosphere, 81.2 mg (0.74 mmol) of hydroquinone, 32.1 mg (0.80 mmol) of sodium hydroxide, and 0.4 mL of 1,3-dimethyl-2-imidazolidinone (DMI) were added sequentially to this powdered carbon fiber reinforced polyether ether ketone (82.4 mg; an amount corresponding to 0.20 mmol of the repeating unit in the general formula on the right side of the chemical reaction formula in Fig. 12), and the obtained reaction mixture was then stirred at 150°C.

After 41 hours, the reaction solution was brought to room temperature (25°C), hydrochloric acid was added, and the precipitated solid mixture was filtered to obtain a crude product. The crude product was washed with water and then dissolved in acetone to extract organic components. The organic components were then dried under reduced pressure to obtain the target synthetic resin decomposition product, bis(4-(4-hydroxyphenoxy)phenyl)methanone (recovery amount 64.8 mg, 0.163 mmol, yield 81%). On the other hand, the black solid obtained by removing the organic components from the above crude product was dried, and 32.1 mg (39 wt%) of carbon fiber powder was recovered.

The ¹H NMR analysis result of the obtained bis(4-(4-hydroxyphenoxy)phenyl)methanone was found to be similar to that in Example 10.

### <Example 12>

Glass fiber reinforced polyether ether ketone (TECAPEEK GF30 manufactured by Ensinger GmbH, Cat. No. 3-3095-01) was processed into powder. In this glass fiber reinforced polyether ether ketone, the content ratio of polyether ether ketone relative to the total mass of the glass fiber reinforced polyether ether ketone was 70 mass %.

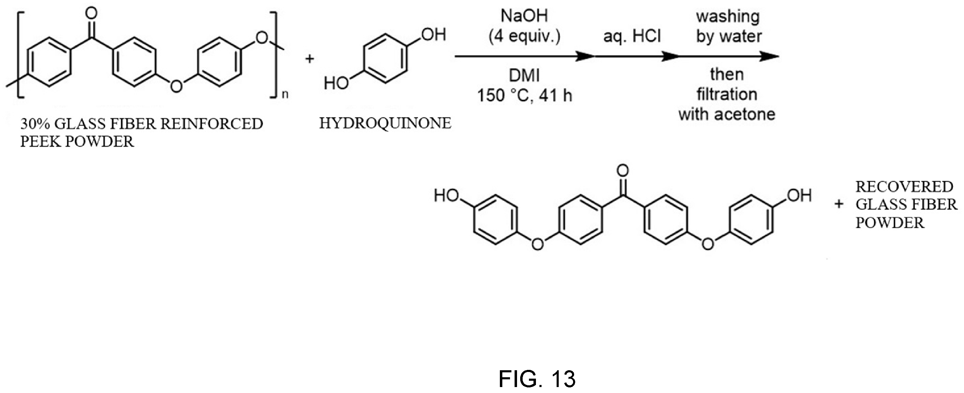

Under a nitrogen atmosphere, 80.7 mg (0.73 mmol) of hydroquinone, 32.0 mg (0.80 mmol) of sodium hydroxide, and 0.4 mL of 1,3-dimethyl-2-imidazolidinone (DMI) were added sequentially to this powdered glass fiber reinforced polyether ether ketone (82.5 mg; an amount corresponding to 0.20 mmol of the repeating unit in the general formula on the right side of the chemical reaction formula in Fig. 13), and the obtained reaction mixture was then stirred at 150°C.

After 41 hours, the reaction solution was brought to room temperature (25°C), hydrochloric acid was added, and the precipitated solid mixture was filtered to obtain a crude product. The crude product was washed with water and then dissolved in acetone to extract organic components. The organic components were then dried under reduced pressure to obtain the target synthetic resin decomposition product, bis(4-(4-hydroxyphenoxy)phenyl)methanone (recovery amount 57.2 mg, 0.144 mmol, yield 72%). On the other hand, the black solid obtained by removing the organic components from the above crude product was dried, and 35.0 mg (42 wt%) of powdered residue containing glass fibers was recovered.

The ¹H NMR analysis result of the obtained bis(4-(4-hydroxyphenoxy)phenyl)methanone was found to be similar to that in Example 10.

As can be seen from the results of Examples 9 to 12, it was found that even when using hydroquinone as the alcohol, synthetic resins were able to be sufficiently decomposed.

### <Example 13>

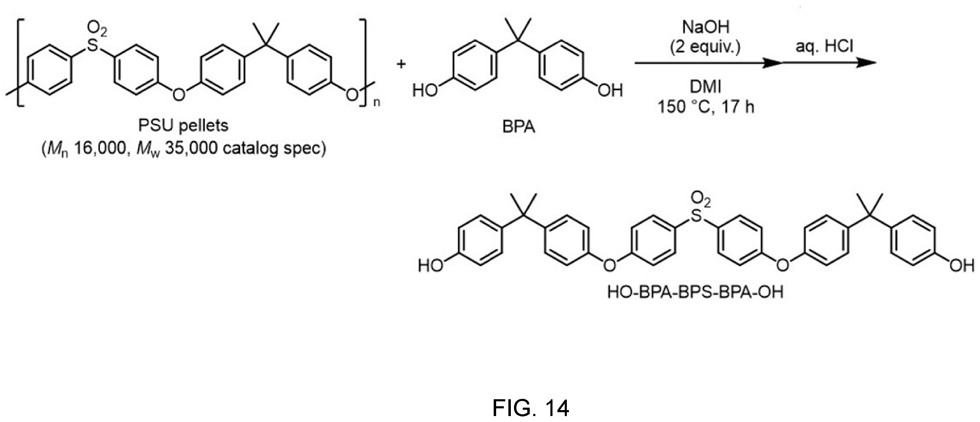

The chemical reaction shown in Fig. 14 was performed as follows.

Under an argon atmosphere, 55.9 mg (0.245 mmol) of bisphenol A, 16.2 mg (0.405 mmol) of sodium hydroxide, and 0.4 mL of 1,3-dimethyl-2-imidazolidinone (DMI) were added sequentially to 89.6 mg of colorless, transparent, pellet-shaped polysulfone (PSU) (0.203 mmol, calculated based on the monomer molar weight, average Mw ~35000, average Mn ~16000, Cat. No. 428302 (manufactured by Sigma-Aldrich Co. LLC)), and the obtained reaction mixture was then stirred at 150°C.

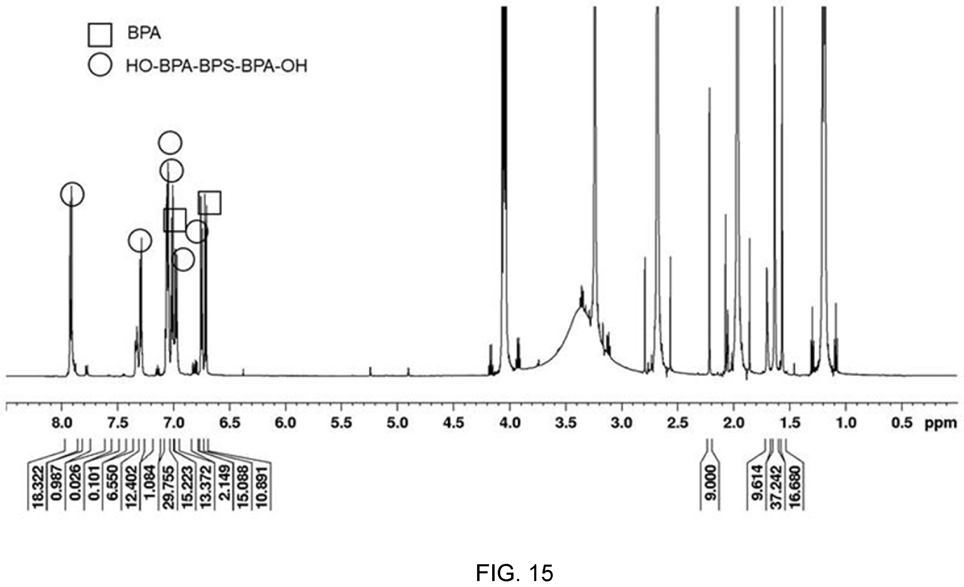

After 17 hours, the yellow, transparent solution was brought to room temperature and ethyl acetate (0.4 mL) and 2 M hydrochloric acid (0.2 mL) were added to separate the layers, and an organic layer was extracted. 5.0 µL of mesitylene as an internal standard was added to this and a small amount was taken out and analyzed by NMR and GC. The analysis showed that 0.11 mmol of the raw material bisphenol A and the target product HO-BPA-BPS-BPA-OH were produced with a yield of 53%. Fig. 15 shows the ¹H NMR analysis result.

### <Example 14>

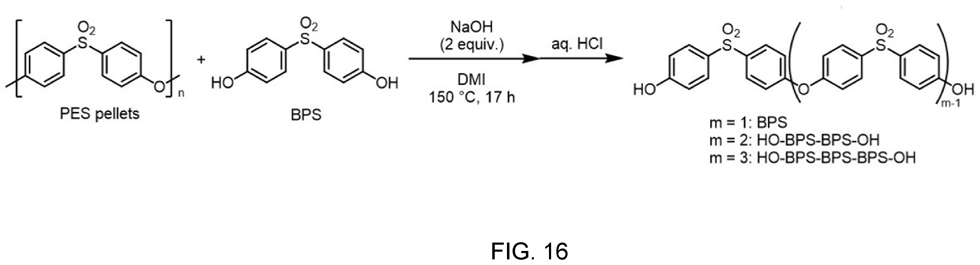

The chemical reaction shown in Fig. 16 was performed as follows.

Under an argon atmosphere, 65.4 mg (0.261 mmol) of bisphenol S, 17.4 mg (0.436 mmol) of sodium hydroxide, and 0.4 mL of 1,3-dimethyl-2-imidazolidinone (DMI) were added sequentially to 50.8 mg of pellet-shaped polyethersulfone (PES) (0.219 mmol, calculated based on the monomer molar weight, Cat. No. 428302 (manufactured by Sigma-Aldrich Co. LLC)), and the obtained reaction mixture was then stirred at 150°C.

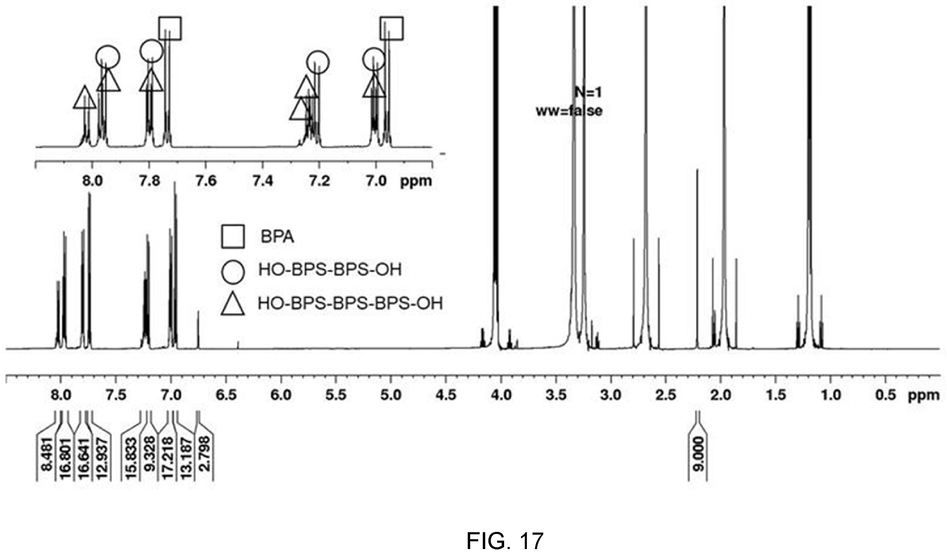

After 17 hours, the yellow, transparent solution was brought to room temperature and ethyl acetate (0.4 mL) and 2 M hydrochloric acid (0.2 mL) were added to separate the layers, and an organic layer was extracted. 5.0 µL of mesitylene as an internal standard was added to this and a small amount was taken out and analyzed by NMR and GC. The analysis showed that 0.12 mmol of the raw material bisphenol S, 0.073 mmol of the product HO-BPS-BPS-OH, and 0.077 mmol of HO-BPS-BPS-BPS-OH were present. Fig. 17 shows the ¹H NMR analysis result.

## Claims

1. A synthetic resin decomposition method for a synthetic resin containing at least one of polysulfone, polyphenylsulfone, polyethersulfone, polyether ether sulfone, and polyether ether ketone, the synthetic resin decomposition method comprising:
a decomposition step for mixing
the synthetic resin,
a base,
an alcohol, the alcohol being at least one of methanol, ethanol, isopropyl alcohol, tert-butyl alcohol, butanol, pentanol, hexanol, heptanol, octanol, nonanol, decanol, 2-ethylhexanol benzyl alcohol, 2-phenylethyl alcohol, phenol, cresol, 4-tert-butylphenol, 4-methoxyphenol, hydroquinone, bisphenol A (2,2-bis(4-hydroxyphenyl)propane), and bisphenol S (bis(4-hydroxyphenyl)sulfone), and
an organic solvent
so as to decompose the polysulfone, the polyphenylsulfone, the polyethersulfone, the polyether ether sulfone, and the polyether ether ketone, contained in the synthetic resin.

2. The synthetic resin decomposition method according to Claim 1, wherein
the base includes at least one of sodium hydroxide, potassium hydroxide, potassium phosphate, cesium hydroxide, cesium carbonate, sodium tert-butoxide, lithium tert-butoxide, potassium tert-butoxide, lithium bis(trimethylsilyl)amide, sodium bis(trimethylsilyl)amide, and potassium bis(trimethylsilyl)amide.

3. The synthetic resin decomposition method according to Claim 1 or 2, wherein
the organic solvent includes at least one of 1,3-dimethyl-2-imidazolidinone, tetramethylurea, 1,3-dimethylpropyleneurea, N-methyl-2-pyrrolidone, N,N-dimethylacetamide, dimethyl sulfoxide, and tetrahydrothiophene-1,1-dioxide.

4. The synthetic resin decomposition method according to Claim 1, wherein a reaction temperature in the decomposition step is within a range of 25°C to 200°C.

5. The synthetic resin decomposition method according to Claim 1, wherein a reaction temperature in the decomposition step is within a range of 25°C to 120°C.

6. The synthetic resin decomposition method according to Claim 1, wherein
the synthetic resin contains at least one of polyether ether ketone, polyether ether sulfone, polysulfone, and polyethersulfone,
the alcohol is at least one of hydroquinone, bisphenol A, and bisphenol S, and
a reaction temperature in the reaction step is within a range of 25°C to 160°C.
